# EUROPEAN PATENT APPLICATION

(11) **EP 2 609 857 A1**
(43) Date of publication of application: **03.07.2013**
(21) Application number: 12199288.7
(22) Date of filing: 21.12.2012
(51) Int. Cl.: A61B 5/08, A61B 5/085, A61B 5/00, A61B 5/113

(54) **Apparatus for detecting an apnea/hypopnea condition**

(30) Priority: 28.12.2011 JP 2011289743
(71) Applicant: Nihon Kohden Corporation, Shinjuku-ku Tokyo (JP)
(72) Inventor: Yamamori, Shinji, Tokyo (JP); Nonaka, Yukio, Tokyo (JP); Uchimasu, Shinichiro, Tokyo (JP)
(74) Representative: Ilgart, Jean-Christophe

(57) **Abstract**

While remarkably reducing the botheration and burden on the subject, an apnea/hypopnea condition can be detected correctly and easily, and the type of the condition can be identified. A detecting apparatus for detecting an apnea/hypopnea condition of a living body, includes: an electric current applying electrode section to supply an electric current via electrodes into a cervical region of the living body; a measurement electrode section to measure an impedance of the cervical region by the electric current; an analysis section which detects an apnea/hypopnea condition based on the measured impedance; and an output section which outputs the result of detection.

## Description

### BACKGROUND OF THE INVENTION

The present invention relates to an apparatus for detecting an apnea/hypopnea condition of the subject.

In a known apparatus of this kind, a temperature sensor is attached to the periphery of the mouth of the subject to detect nasal and oral respiration, and a strap is wound around the chest and abdominal regions of the subject to detect motions of the regions caused by the respiratory motion. Then, identification is performed based on a combination of detection results of the sensors, so that an apnea/hypopnea condition is detected and the types of the condition is identified. The types of an apnea/hypopnea condition is roughly classified into two categories, namely, obstructive apnea/hypopnea in which a respiratory effort is continued in an airway obstruction condition, and central apnea/hypopnea in which a respiratory effort itself stops to cease the ventilation.

Patent Reference 1 discloses an evaluation apparatus which processes a measurement signal indicative of the respiratory gas flow of the patient, to enable obstructive respiratory disorder and central respiratory disorder to be distinguished from each other. Patent Reference 2 discloses a condition analysis apparatus in which the respiratory condition of an object is identified by comparing measurement data that are obtained in the chest or abdominal region of the object, with a threshold.

### [Prior Art Reference]

### [Patent Reference]

[Patent Reference 1] Japanese Patent NO. 4,478,027
[Patent Reference 2] Japanese Patent NO. 4,588,461

In order to diagnose sleep apnea syndrome, a polysomnography test is performed in which, in addition to the above-described biological signals, movement patterns of brain waves and the eye balls, an electrocardiogram, muscle movements, the blood oxygen saturation, the volume of snoring, and the like are measured overnight. The sensor which is disclosed in Patent Reference 1, and which is attached to the periphery of the nose or the mouth in order to detect a change of the gas flow due to nasal and oral respiration inevitably causes the subject to feel bothersome. The strap which is disclosed in Patent Reference 2, and which is wound around the chest and abdominal regions must be tightly wound in order to enhance the measurement accuracy. This imposes a large burden on the subject. These botheration and burden may sometimes impede the measurement.

On the other hand, a method called the impedance respiration has been known. In the measuring method, a part of electrodes for measuring an electrocardiogram is used, and the respiratory condition is detected from an impedance change due to respiration in the chest and abdominal regions (hereinafter, generally referred to as the trunk region). According to the measuring method, the burden and botheration on the patient can be remarkably reduced. However, the method has a problem in that, in an obstructive apnea or hypopnea condition, an impedance change which is caused by a respiratory effort is often erroneously recognized as usual respiration.

### SUMMARY

This invention provides a technique in which, while remarkably reducing the botheration and burden on the subject, an apnea/hypopnea condition is detected correctly and easily, and the types of the condition is identified.

It is therefore an aspect of the invention to provide a detecting apparatus for detecting an apnea/hypopnea condition of a living body, including:
an electric current applying electrode section configured to supply an electric current via electrodes into a cervical region of the living body;
a measurement electrode section configured to measure impedance of the cervical region by the electric current;
an analysis section configured to detect an apnea/hypopnea condition based on the measured impedance; and
an output section configured to output the result of detection.

According to the configuration, an impedance change is measured in the cervical region in which a noticeable motion is caused by a respiratory effort performed in an airway obstruction condition, and therefore an obstructive apnea/hypopnea condition can be detected without causing the subject to feel substantial botheration and pain.

The apparatus may further include a respiration sensor which is placed on a region of the living body other than the cervical region, and configured to output a signal caused by respiration or respiratory effort of the living body, and
the analysis section may be configured to identify types of the apnea/hypopnea condition based on the measured impedance and the output signal of the respiration sensor.

The output section may be configured to visualize the identification result.

According to the configuration, the accuracy of detection of an obstructive apnea/hypopnea condition can be improved, and furthermore the condition can be distinguished from a central apnea/hypopnea condition.

The analysis section may be configured to identify whether the living body is in an obstructive apnea/hypopnea condition or a central apnea/hypopnea condition, based on the measured impedance and the output signal of the respiration sensor.

The respiration sensor may be a sensor which measures respiration related impedance variation in the trunk region of the living body, and
the analysis section,
in the case where both the measured impedance and the output signal of the respiration sensor are equal to or larger than respective thresholds, may be configured to identify that the living body is in an obstructive apnea/hypopnea condition, and,
in the case where both the measured impedance and the output signal of the respiration sensor are smaller than the respective thresholds, may be configured to identify that the living body is in a central apnea/hypopnea condition.

In the configuration where the respiration related impedance variation of the trunk region is measured by sharing electrodes with an electrocardiogram which are used also in a polysomnography, particularly, the number of sensors to be attached can be minimized, and therefore the burden on the subject can be suppressed.

Each of the electric current applying electrode section and the measurement electrode section includes at least one pair of electrodes respectively. Furthermore, both one electrode of the electrodes pair of the electric current applying electrode section and one electrode of the electrodes pair of the measurement electrode section are placed on a common supporting component.

In this case, attachment to the cervical region can be performed more smoothly, and the distance between the electrodes can be set to be constant, so that the potential measurement is further stabilized.

The apparatus may be configured so that one pair of electrodes is configured to function as both the electric current applying electrode section and the measurement electrode section. In this case, the user is requested to attach only the one pair of electrodes to the living body, and therefore the measurement can be simply performed.

### BRIEF DESCRIPTION OF THE DRAWINGS

Fig. 1 is a functional block diagram showing a detecting apparatus of a first embodiment of the invention.
Fig. 2 is a view diagrammatically showing an arrangement example in the case where an electrode group of the detecting apparatus of Fig. 1 is attached to the living body.
Fig. 3 is a view showing an example of a waveform which is obtained by a measurement section of the detecting apparatus of Fig. 1.
Fig. 4 is a functional block diagram showing a detecting apparatus of a second embodiment of the invention.
Fig. 5 is a view showing examples of waveforms which are obtained by a measurement section of the detecting apparatus of Fig. 4.
Fig. 6 is a table showing references for determining the types of an apnea condition in the detecting apparatus of Fig. 4.
Figs. 7A to 7C are views diagrammatically showing arrangement examples of the electrode group of the detecting apparatus of Fig. 1.
Figs. 8A and 8B are views diagrammatically showing modifications of the electrode group of the detecting apparatus of Fig. 1.
Fig. 9A is a functional block diagram showing a modification of the detecting apparatus of Fig. 1, and Fig. 9B is a view diagrammatically showing an arrangement example of an electrode group.

### DETAILED DESCRIPTION OF EMBODIMENTS

Hereinafter, embodiments of the invention will be described in detail with reference to the accompanying drawings.

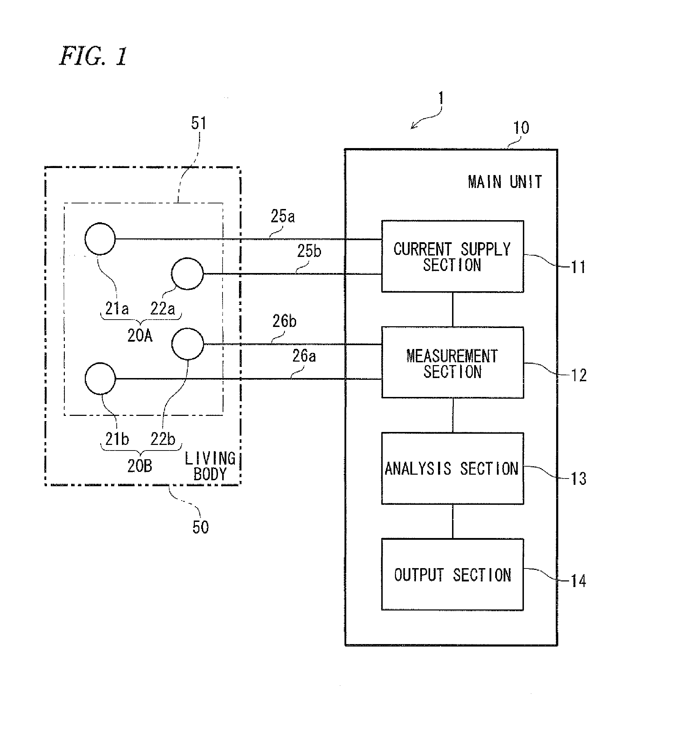

Fig. 1 is a functional block diagram showing a detecting apparatus 1 of a first embodiment of the invention. The detecting apparatus 1 detects an apnea/hypopnea condition of the subject, and includes the main unit 10 and an electrode group to be attached to the cervical region 51 of the subject (living body) 50.

The main unit 10 includes a current supply section 11, a measurement section 12, an analysis section 13, and an output section 14. The electrode group includes an electric current applying electrode section 20A and a measurement electrode section 20B. The electric current applying electrode section 20A is configured by a first electric current applying electrode 21a and a second electric current applying electrode 22a, and the measurement electrode section 20B by a first measurement electrode 21b and a second measurement electrode 22b.

The current supply section 11 is electrically connected to the first electric current applying electrode 21a and the second electric current applying electrode 22a through respective signal lines 25a, 25b. In the embodiment, a current of from 10 µA to 1 mA (peak-to-peak value) and from 10 to 100 kHz is supplied from the current supply section 11 to the first electric current applying electrode 21a and the second electric current applying electrode 22a through the signal lines 25a, 25b.

The first measurement electrode 21b and the second measurement electrode 22b are electrically connected to the measurement section 12 through signal lines 26a, 26b, respectively. The first measurement electrode 21b and the second measurement electrode 22b supply the potentials of the respective attached portions to the measurement section 12 through the signal lines 26a, 26b.

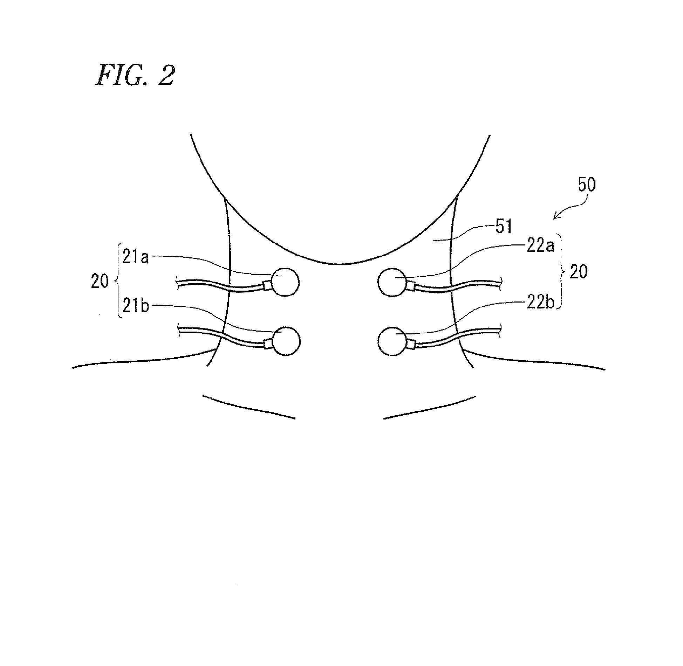

As shown in Fig. 2, the electrode group is attached to the cervical region 51 of the subject 50. The measurement section 12 measures the impedance of the cervical region 51 based on the values of the currents supplied to the first electric current applying electrode 21a and the second electric current applying electrode 22a, and the potential difference arisen between the first measurement electrode 21b and the second measurement electrode 22b.

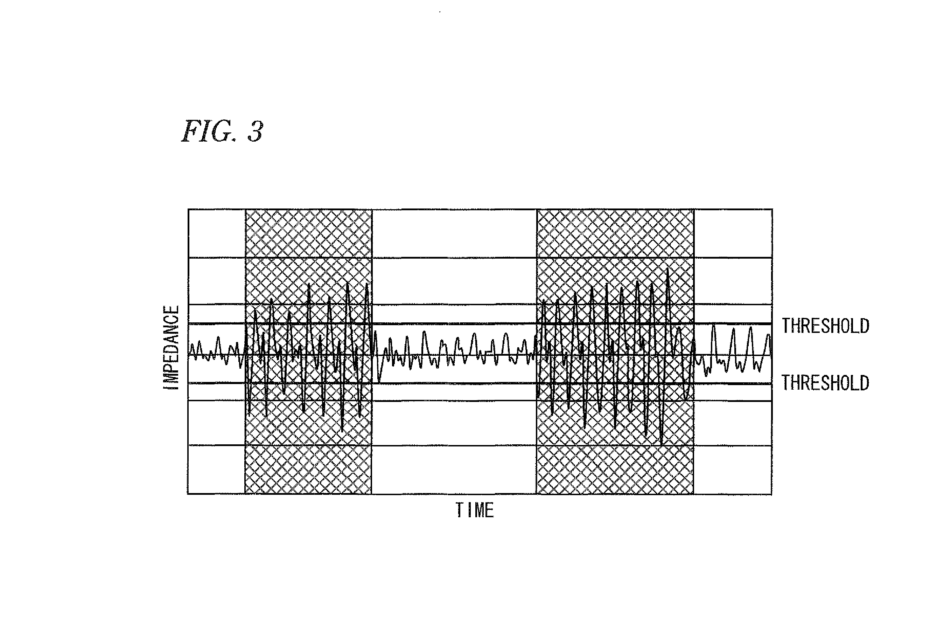

The waveform shown in Fig. 3 indicates a change with time of the impedance of the cervical region 51 which is measured by the measurement section 12. The abscissa of the graph indicates the measurement time in a polysomnography. In the case of obstructive apnea/hypopnea, when a respiratory effort is performed in an airway obstruction condition, the pressure in the airway is usually negative during inspiration, and hence the pharynx is pulled toward the stomach, thereby causing the value of the impedance of the cervical region 51 to which the electrode group 20 is attached, to largely change.

In the detecting apparatus 1 of the embodiment, attention is focused on the fact that the change of the impedance value can indicate an obstructive apnea/hypopnea condition in sleep apnea syndrome. The detecting apparatus is configured so that an apnea/hypopnea condition can be detected based on a change of the impedance of the cervical region 51 which is measured by the measurement section 12. Specifically, the analysis section 13 is communicatably connected to the measurement section 12, and configured so that, when the measured impedance exceeds a predetermined threshold, it is identified that the subject is in an obstructive apnea/hypopnea condition.

Alternatively, the analysis section 13 may be configured so that, when the amplitude of the measured impedance which changes with time exceeds a predetermined threshold, it is identified that the subject is in an obstructive apnea/hypopnea condition. In the subsequent description, the term "the measured impedance" means to include both the absolute and amplitude values of the impedance. The thresholds of the measured value and the amplitude may be predetermined constant values, or changeable in accordance with the subject or the measurement environment.

The output section 14 is communicatably connected to the analysis section 13, and configured so as to output detection results of an apnea/hypopnea condition with a visualization technique or the like. The output technique may be performed in any one of various modes such as an audio output, printing by a printer, and data transmission to a predetermined apparatus.

As shown in Fig. 3, for example, lines indicating thresholds are displayed, so that a medical person can easily recognize in a visual manner that the measured impedance exceeds a threshold. When an obstructive apnea/hypopnea condition occurs in attend (overnight monitoring) medical care, for example, it is possible to immediately check the condition and other parameters of the patient.

In addition to or in place of this, for example, a time zone when a measured impedance value which exceeds a threshold is obtained is indicated by changing the color of the background as indicated by the hatched portion in Fig. 3, so that time zone when an apnea/hypopnea condition occurs can be made recognizable. According to this technique, for example, it is possible to easily and intuitively find a time zone when an apnea condition occurs, after the end of the test or the like.

The detection result of an apnea/hypopnea condition which has been visualized as described above is output by displaying the result on a display device such as a display monitor (not shown). Alternatively, the result may be output by printing the result on a predetermined sheet by using a printing mechanism which is not shown. Each of the display device and the printing mechanism may be accommodated in the main unit 10 of the detecting apparatus 1, or in an external apparatus which is communicatably connected to the main unit 10.

In the embodiment, as described above, an apnea/hypopnea condition is detected by the electrode group 20 which is attached to the cervical region 51 of the subject 50, and therefore it is not required that a sensor is placed in the periphery of the mouth of the subject, or that a strap is tightly wound around the chest and abdominal regions. In a polysomnography test, therefore, the respiratory condition can be detected while remarkably reducing botheration and pain of the subject.

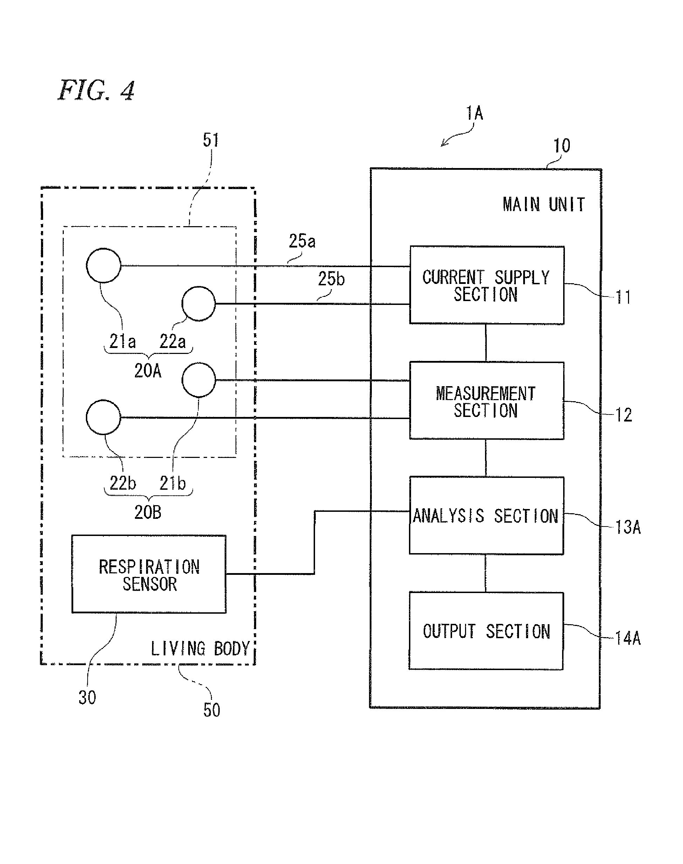

Next, a detecting apparatus 1A of a second embodiment of the invention will be described with reference to Fig. 4. The components which are substantially identical with or similar to those of the first embodiment are denoted by the same reference numerals, and duplicated description is omitted.

The detecting apparatus 1A of the embodiment is different from the above-described detecting apparatus 1 in that the apparatus includes a respiration sensor 30 which is placed in a region other than the cervical region, and which outputs a signal corresponding to respiration of the subject, and also that an analysis section 13A identifies the types of an apnea/hypopnea condition, based on the measured impedance of the cervical region 51, and an output signal from the respiration sensor.

The respiration sensor 30 in the embodiment is configured by groups of electrodes which are used for measuring an electrocardiogram (hereinafter, such an electrode is referred to as "electrocardiogram measuring electrode"), and which are used also in a polysomnography test, and measures the impedance respiration of the trunk region of the subject 50, based on the potential difference arisen between the electrode groups. The impedance respiration of the trunk region changes in accordance with the respiratory motion (and a respiratory effort). Therefore, the impedance can be obtained as an output of a signal corresponding to respiration of the subject 50. The measurement itself of the impedance respiration of the trunk region by using groups of electrocardiogram measuring electrodes is a known technique, and therefore its detailed description is omitted.

The analysis section 13A is communicatably connected to the measurement section 12 and the respiration sensor 30, and configured so that detection of an apnea/hypopnea condition and identification of the types of the condition are performed based on the measured impedance of the cervical region 51 of the subject 50 which is measured by the measurement section 12, and that of the impedance respiration of the trunk region of the subject 50 which is measured by the respiration sensor 30.

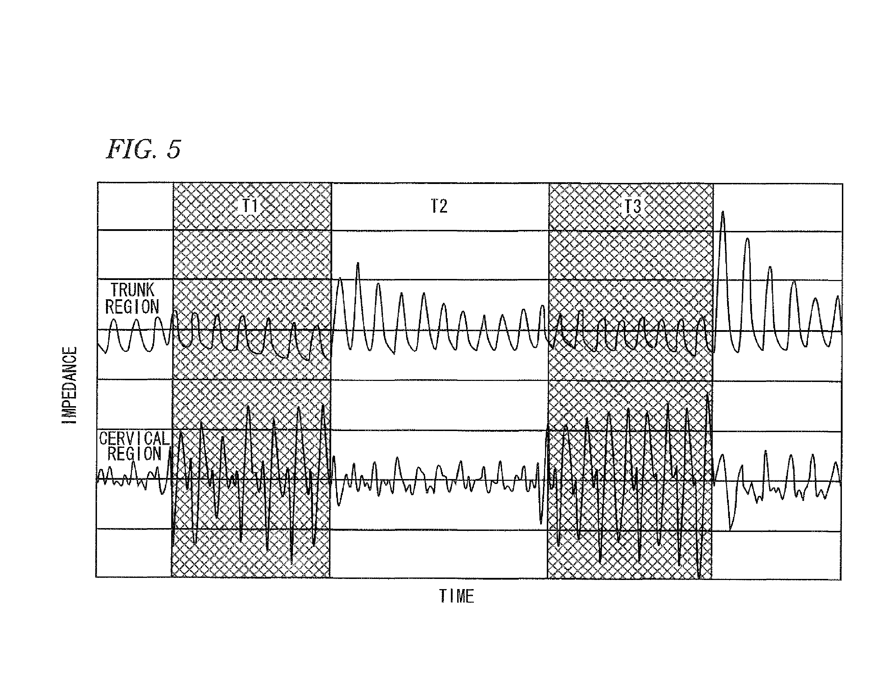

The waveforms shown in Fig. 5 indicate changes with time of the impedance of the cervical region which is measured by the measurement section 12, and that of the trunk region which is measured by the electrocardiogram measuring electrodes. The abscissa of the graph indicates the measurement time in a polysomnography, and the ordinate indicates the impedance. The illustration in which the impedance change of the cervical region and the measurement waveform of the impedance respiration of the trunk region are vertically arranged is intended to display them in temporal synchronization with each other, and does not show their absolute level relationship between the measured waveforms.

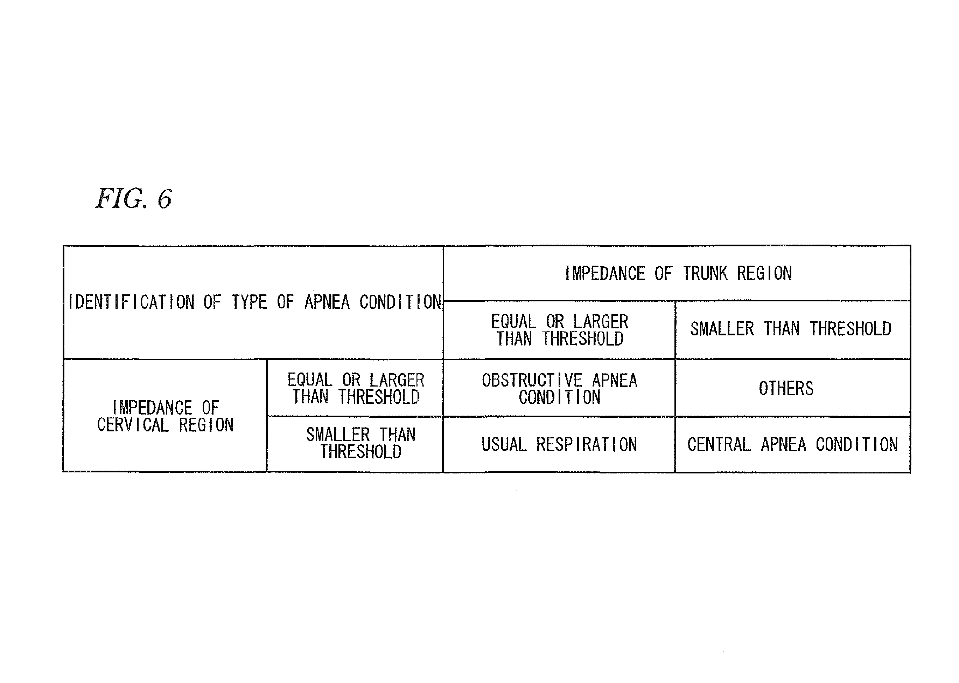

Fig. 6 is a table showing references used in the case where the analysis section 13A identifies the types of an apnea/hypopnea condition. When the measured impedance of the cervical region exceeds a predetermined threshold and that of the impedance respiration of the trunk region exceeds a predetermined threshold, the analysis section 13A identifies that the subject is in an obstructive apnea/hypopnea condition.

An obstructive apnea/hypopnea condition means a condition in which a respiratory effort is performed in an airway obstruction condition or an airway constriction condition. Therefore, a change occurs in the impedance which is measured by the groups of electrocardiogram measuring electrodes attached to the trunk region, and a change which, as described in the first embodiment, is larger than that during normal respiration occurs in the impedance which is measured by the electrode group 20 attached to the cervical region 51.

In Fig. 5, the waveforms of the time periods T1 and T3 show the condition. Because of a respiratory effort at these time periods, a change which is similar to that obtained as if the subject respires normally is observed in the impedance respiration of the trunk region, and, by contrast, an impedance change of an amplitude which is obviously larger than that obtained during normal respiration appears in the cervical region.

In the case where the measured impedance of the cervical region is smaller than the predetermined value, and that of the impedance respiration of the trunk region is equal to or larger than the predetermined value, as shown in Fig. 6, the analysis section 13A identifies that the subject is in a normal respiration condition.

As against the time period T1 when the airway is closed or constricted and the subject is in an apnea/hypopnea condition, the airway is released in the time period T2. During apnea/hypopnea, usually, the subject is lack of oxygen. Immediately after release of the airway, therefore, the ventilation is often largely performed in order to solve the lack of oxygen. Even immediately after such apnea, the airway is opened, and therefore the impedance change of the cervical region 51 due to respiration (effort) is obviously smaller than that during when the airway is closed or constricted. By contrast, in the impedance respiration of the trunk region, as the degree of respiration (effort) is higher, usually, the amplitude tends to be larger in both the case where the airway is released and respiration is normally performed, and the case where the airway is closed. The waveforms shown in the time period T2 in Fig. 5 indicate this state.

As described above, an obstructive apnea/hypopnea condition is hardly detected based on only the measurement waveform of the impedance respiration of the trunk region shown in the time periods T1 and T3 in Fig. 5. In the embodiment, the impedance of the cervical region which is remarkably changed in an obstructive apnea/hypopnea condition is referred, so that the obstructive apnea/hypopnea condition can be recognized easily and surely.

As shown in Fig. 6, in the case where the measured impedance of the cervical region is smaller than the predetermined threshold, and that of the impedance respiration of the trunk region is equal to or smaller than the predetermined threshold, the analysis section 13A identifies that the subject is in a central apnea/hypopnea condition.

A central apnea/hypopnea condition means a condition in which a command with respect to respiration is not normally transmitted from respiratory center, and the respiratory motion itself is not performed. Therefore, a large change does not occur in both the impedance which is measured by the electrode group 20 attached to the cervical region 51, and the impedance respiration which is measured by the groups of electrocardiogram measuring electrodes attached to the trunk region. Consequently, both the measurement waveforms of the impedance of the cervical region and the impedance respiration of the trunk region are approximately flat, and are recognized as measured values which are smaller than the respective thresholds.

An output section 14A is communicatably connected to the analysis section 13A, and configured so as to visualize and output results of identification of an apnea condition. The visualization and output techniques are similar to those of the output section 14 in the first embodiment, and the display is performed so as to exhibit an appearance such as shown in Fig. 5. An obstructive apnea/hypopnea condition and a central apnea/hypopnea condition can be distinguished from each other by differentiating the colors of the backgrounds on which the conditions change. In addition to or in place of this, symbols or the like each indicating that an obstructive or central apnea/hypopnea occurs may be displayed so that the two conditions can be distinguished from each other.

As described above, according to the embodiment, when combined with the well-known method of measuring the impedance respiration of the trunk region, not only detection of an obstructive apnea/hypopnea condition can be performed easily and surely, but also discrimination of the condition from a central apnea/hypopnea condition is enabled. In the measurement of the impedance respiration of the trunk region, when electrocardiogram measuring electrodes which are used also in a polysomnography test are used, the number of sensors to be attached can be minimized, and therefore the burden on the subject can be suppressed.

The embodiments have been described in order to facilitate understanding of the invention, and are not intended to limit the invention. It is a matter of course that the invention may be changed or improved without departing the spirit thereof, and includes equivalents thereof.

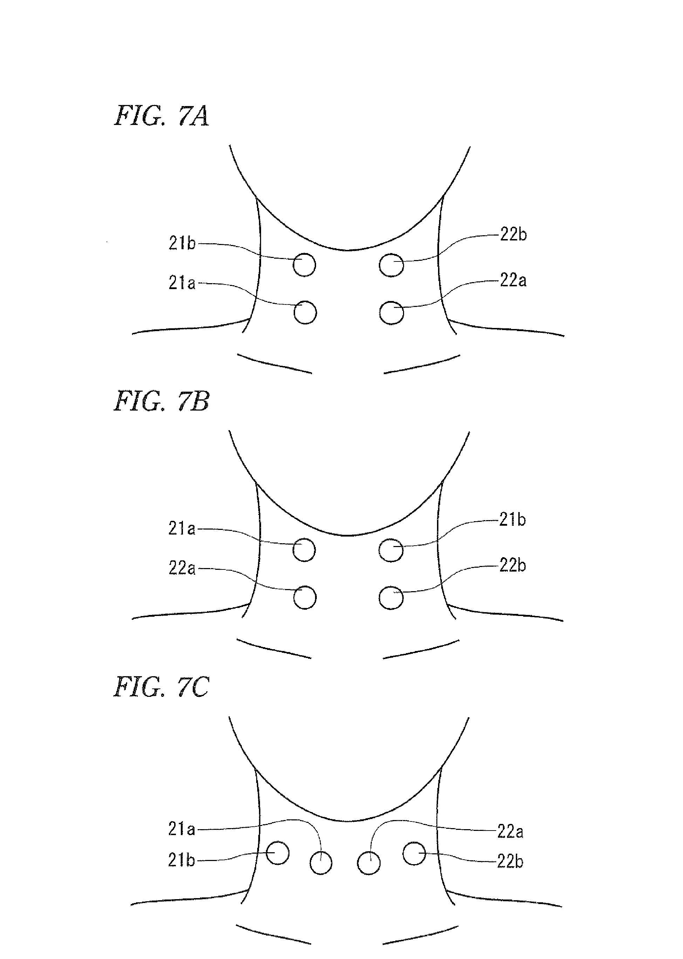

The positions where the electrode group 20 is attached to the cervical region 51 are not limited to those shown in Fig. 2. As far as the potential difference between two points in the cervical region 51 can be measured, any one of appropriate arrangements shown in Figs. 7A to 7C may be selected.

As shown in Fig. 7A, in a manner opposite to the arrangement example shown in Fig. 2, for example, the electric current applying electrodes 21a, 22a may be placed in the lower side, and the measurement electrodes 21b, 22b may be placed in the upper side. As shown in Fig. 7B, alternatively, the electric current applying electrodes 21a, 22a may be placed on the right side of the body, and the measurement electrodes 21b, 22b may be placed on the left side of the body, or the arrangement opposite to this may be employed. As shown in Fig. 7c, furthermore, the electrodes may be laterally arranged in one row, the electric current applying electrodes 21a, 22a may be placed in the inner side, and the measurement electrodes 21b, 22b may be placed in the outer side, or the arrangement opposite to this may be employed.

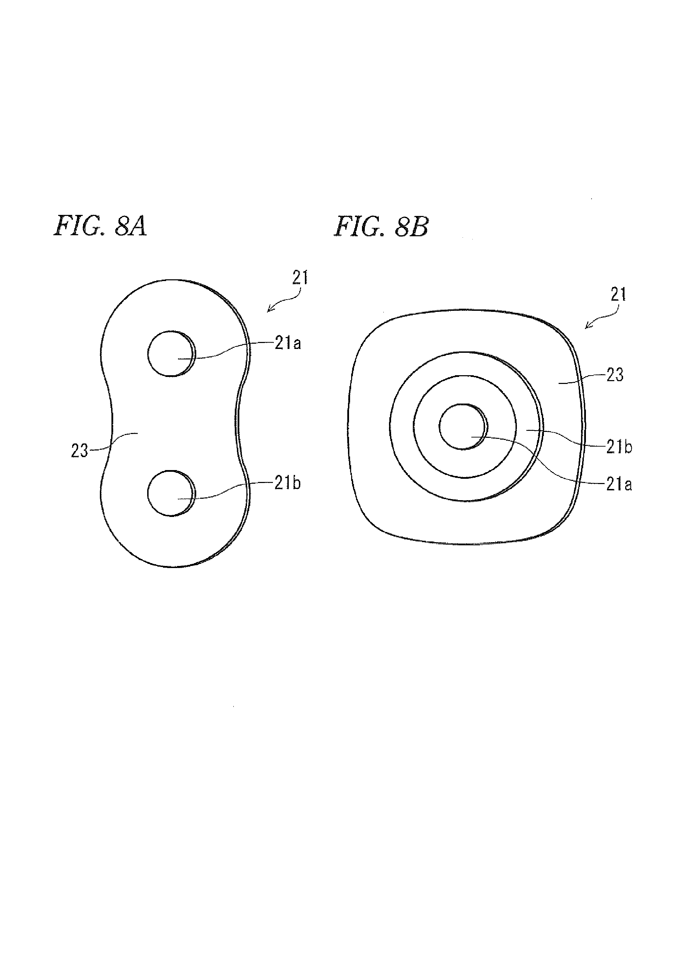

As shown in Fig. 8A, a configuration may be employed in which the first electric current applying electrode 21a and the first measurement electrode 21b are supported by a common support member 23. As shown in Fig. 8B, another configuration may be employed in which the first electric current applying electrode 21a and the first measurement electrode 21b are concentrically arranged on the common support member 23. The above is applicable also to the second electric current applying electrode 22a and the second measurement electrode 22b.

According to the above configurations, the electrodes can be attached more smoothly to the cervical region 51, and the distance between the electrodes is constant. Therefore, the stability of the potential measurement is enhanced. This is applicable also to the second electric current applying electrode 22a and the second measurement electrode 22b.

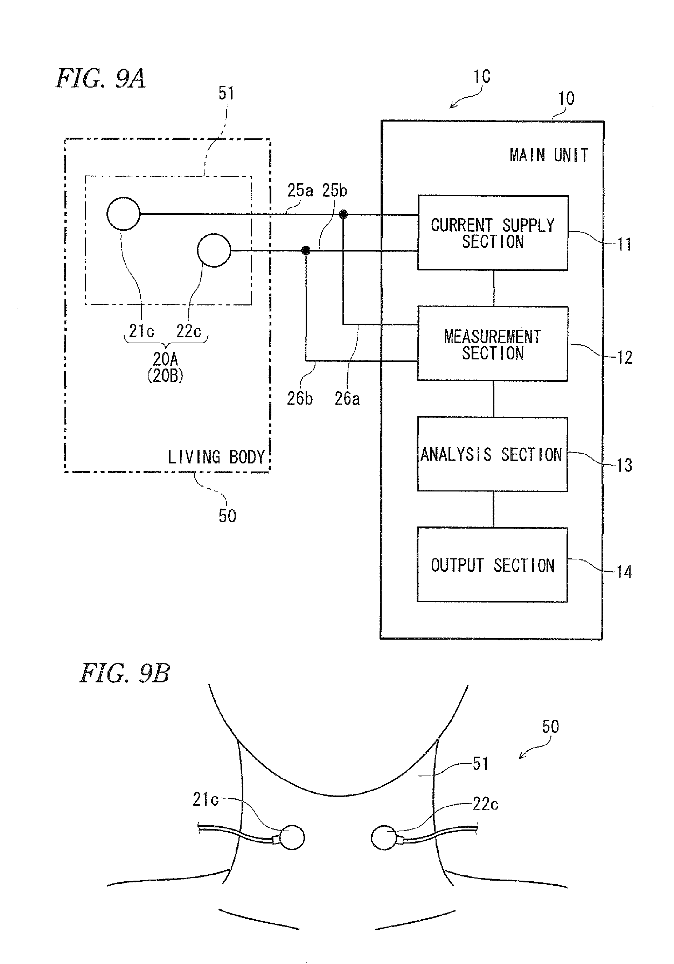

As shown in Fig. 9A, a configuration may be employed in which one pair of electrodes 21c, 22c functions as the electric current applying electrode section 20A and the measurement electrode section 20B. In this case, the signal lines 26a, 26b connected to the measurement section 12 are connected in parallel, respectively, to the pair of electrodes 21c, 22c, and, as shown in Fig. 9B, the pair of electrodes 21c, 22c is attached to the cervical region 51 of the subject 50. According to the configuration, the user is requested to attach only the one pair of electrodes to the subject, and therefore an easier measurement is enabled.

Alternatively, a configuration may be employed in which each of the electric current applying electrode section 20A and the measurement electrode section 20B includes a plurality of pairs of electrodes.

As far as the respiration sensor 30 can measure the impedance respiration of the trunk portion of the subject 50, the respiration sensor is not necessary to use electrocardiogram measuring electrodes. A well-known sensor for measuring the impedance respiration can be adequately used.

When the respiration sensor 30 is placed in a region of the subject 50 other than the cervical region 51, and can output a signal corresponding to respiration of the subject 50, it is not always necessary to use a sensor for measuring the impedance respiration. For example, a flow sensor which can detect nasal and oral respiration of the subject 50 may be used.

According to the configuration of the invention, while remarkably reducing the botheration and burden on the subject, an apnea/hypopnea condition can be detected correctly and easily, and the types of the condition can be identified.

## Claims

1. A detecting apparatus for detecting an apnea/hypopnea condition of a living body, including:
an electric current applying electrode section configured to supply an electric current via electrodes into a cervical region of the living body;
a measurement electrode section configured to measure impedance of the cervical region by the electric current;
an analysis section configured to detect an apnea/hypopnea condition based on the measured impedance; and
an output section configured to output the result of detection.

2. The detecting apparatus according to claim 1, wherein the detecting apparatus further includes a respiration sensor which is placed on a region of the living body other than the cervical region, and configured to output a signal caused by respiration or respiratory effort of the living body, and
the analysis section is configured to identify types of the apnea/hypopnea condition based on the measured impedance and the output signal of the respiration sensor.

3. The detecting apparatus according to claim 2, wherein the output section is configured to visualize the identification result.

4. The detecting apparatus according to claim 2, wherein the analysis section is configured to identify whether the living body is in an obstructive apnea/hypopnea condition or a central apnea/hypopnea condition, based on the measured impedance and the output signal of the respiration sensor.

5. The detecting apparatus according to any one of claims 2 to 4, wherein the respiration sensor is a sensor which measures respiration related impedance variation in the trunk region of the living body, and
the analysis section,
in the case where both the measured impedance and the output signal of the respiration sensor are equal to or larger than respective thresholds, is configured to identify that the living body is in an obstructive apnea/hypopnea condition, and,
in the case where both the measured impedance and the output signal of the respiration sensor are smaller than the respective thresholds, is configured to identify that the living body is in a central apnea/hypopnea condition.

6. The detecting apparatus according to claim 5, wherein the respiration related impedance variation of the trunk region is measured by sharing electrodes with an electrocardiogram.

7. The detecting apparatus according to any one of claims 1 to 6, wherein each of the electric current applying electrode section and the measurement electrode section includes at least one pair of electrodes respectively.

8. The detecting apparatus according to claim 7, wherein both one electrode of the electrodes pair of the electric current applying electrode section and one electrode of the electrodes pair of the measurement electrode section are placed on a common supporting component.

9. The detecting apparatus according to any one of claims 1 to 6, wherein one pair of electrodes is configured to function as both the electric current applying electrode section and the measurement electrode section.
